Europäisches Patentamt

European Patent Office

Office européen des brevets

(19)

(11) Veröffentlichungsnummer: **0 328 959**
**A2**

(12) **EUROPÄISCHE PATENTANMELDUNG**

(21) Anmeldenummer: **89101866.5**

(51) Int. Cl.4: **C07H 15/04**

(22) Anmeldetag: **03.02.89**

(30) Priorität: **13.02.88 DE 3804599**

(43) Veröffentlichungstag der Anmeldung:
**23.08.89 Patentblatt 89/34**

(84) Benannte Vertragsstaaten:
**BE DE ES FR GB IT NL**

(71) Anmelder: **BASF Aktiengesellschaft**
**Carl-Bosch-Strasse 38**
**D-6700 Ludwigshafen(DE)**

(72) Erfinder: **Baur, Richard, Dr.**
**Nelkenstrasse 1**
**D-6704 Mutterstadt(DE)**
Erfinder: **Houben, Jochen, Dr.**
**Schulstrasse 9-17**
**D-6700 Ludwigshafen(DE)**
Erfinder: **Jaeger, Hans-Ulrich, Dr.**
**Erschigweg 31**
**D-6730 Neustadt(DE)**
Erfinder: **Oftring, Alfred, Dr.**
**Im Roehrich 49**
**D-6702 Bad Duerkheim(DE)**
Erfinder: **Perner, Johannes, Dr.**
**Ginsterweg 4**
**D-6730 Neustadt(DE)**

(54) **Verfahren zur Reinigung von langkettigen Alkylglucosiden.**

(57) Verfahren zur Reinigung von langkettigen Alkylglucosiden durch Extraktion der langkettigen Alkylglucoside aus Reaktionsgemischen, die durch Umsetzung von Monosacchariden und mindestens 8 C-
Atome enthaltenden Alkoholen oder durch Umacetalisierung von kurzkettigen Alkylglucosiden mit mindestens 8 C-Atome aufweisenden Alkoholen erhältlich und die jeweils auf einen pH-Wert von oberhalb
6 eingestellt sind, mit Hilfe von Wasser.

EP 0 328 959 A2

## Verfahren zur Reinigung von langkettigen Alkylglucosiden

Langkettige Alkylglucoside, d.h. Alkylglucoside, deren Alkylkette mindestens 8 Kohlenstoffatome aufweist, werden in der Technik als nichtionische Tenside mit gutem Netzvermögen verwendet, z.B. in hoch alkalischen Industriereinigern. Sie werden in zunehmendem Maße auch als Bestandteil in Haut- und Haarpflegemitteln eingesetzt. Hierfür sind jedoch besonders reine Produkte erforderlich.

Langkettige Alkylglucoside werden beispielsweise durch Umsetzung von Monosacchariden oder zu Monosacchariden hydrolysierbaren reduzierend wirkenden Zuckern und mindestens 8 C-Atome enthaltenden Alkoholen in Gegenwart saurer Katalysatoren hergestellt. Man kann jedoch auch kurzkettige Alkylglucoside, z.B. $C_1$-$C_6$-Alkylglucoside in Gegenwart von sauren Katalysatoren mit mindestens 8 C-Atome aufweisenden Alkoholen umacetalisieren. Bei beiden Herstellungsvarianten der langkettigen Alkylglucoside benötigt man jedoch immer einen erheblichen Überschuß an langkettigen Alkoholen. Man erhält also immer Reaktionsmischungen, die aus dem langkettigen Alkylglucosid und dem überschüssigen langkettigen Alkohol bestehen. Das langkettige Alkylglucosid muß aus diesen Mischungen gewonnen werden, weil anderenfalls bei der Anwendung der langkettigen Alkylglucoside in wässrigen Medien keine klaren Lösungen entstehen. Verfahren zur Herstellung von langkettigen Alkylglucosiden sind bekannt, vgl. beispielsweise US-PS 3 219 656, US-PS 3 598 865, US-PS 3 839 318 und US-PS 3 547 828.

Um die langkettigen Alkylglucoside aus den Reaktionsmischungen zu isolieren, hat man bisher die langkettigen Alkohole daraus unter stark vermindertem Druck und bei Temperaturen um etwa 140°C abdestilliert. Aufgrund der hohen thermischen Belastungen der Alkylglucoside kommt es zu Verharzung und vor allem bei den längerkettigen Alkylglucosiden zunehmend zur Bildung dunkel gefärbter Produkte.

Aus der US-PS 3 547 828 ist zur Reinigung von neutralisierten Mischungen aus langkettigen Alkylglucosiden und langkettigen Alkoholen bekannt, die Mischungen mit Aceton zu behandeln. Eine solche Reinigungsmethode ist technisch aber nur mit einem sehr hohen Kostenaufwand zu bewerkstelligen. Aus der DE-OS 30 01 064 ist ein Verfahren zur Reinigung von $C_8$- bis $C_{16}$-Alkylglucosiden bekannt, die durch Reaktion von kurzkettigen Alkylglucosiden oder Hydroxyalkylglucosiden mit $C_8$- bis $C_{16}$-Alkoholen in Gegenwart saurer Katalysatoren erhalten werden. Bei diesem Verfahren wird der langkettige Alkohol nach einer Neutralisation der Reaktionsmischung destillativ abgetrennt, wobei man die destillative Abtrennung zumindest der letzten Anteile an nicht umgesetzten langkettigen Alkoholen in Gegenwart von Glykolen durchführt, deren Siedepunkte höchtens 10°C oberhalb oder höchstens 30°C unterhalb der Siedepunkte der abzutrennenden langkettigen Alkohole liegen. Der Zusatz der Glykole bewirkt zwar eine Verminderung der Viskosität der durch Destillation zu reinigenden Mischung, erfordert aber andererseits wieder das Entfernen der zugesetzten Glykole. Aus der US-PS 3 839 318 ist außerdem bekannt, aus Mischungen von langkettigen Alkoholen und langkettigen Alkylglucosiden den überschüssigen langkettigen Alkohol mit Hilfe eines Lösemittels aus der Mischung abzutrennen. Als Lösemittel wird beispielsweise Heptan verwendet. Dieses Verfahren hat jedoch den Nachteil, daß es zusätzlich den Einsatz eines Lösemittels erfordert, das wieder zurückgewonnen werden muß.

Der Erfindung liegt die Aufgabe zugrunde, ein schonendes Verfahren zur Reinigung von langkettigen Alkylglucosiden zur Verfügung zu stellen, das kostengünstiger und mit geringerem technischen Aufwand durchführbar ist als die bisher bekannten Verfahren.

Die Aufgabe wird erfindungsgemäß gelöst mit einem Verfahren zur Reinigung von langkettigen Alkylglucosiden durch Extraktion von Reaktionsgemischen, die durch Umsetzung von Monosacchariden und mindestens 8 C-Atome enthaltenden Alkoholen oder durch Umacetalisierung von $C_1$- bis $C_6$-Alkylglucosiden mit mindestens 8 C-Atome aufweisenden Alkoholen erhältlich und die jeweils auf einen pH-Wert oberhalb von 6 eingestellt sind, wenn man die langkettigen Alkylglucoside mit Hilfe von Wasser aus den Reaktionsgemischen extrahiert. Die Extraktion kann kontinuierlich oder diskontinuierlich durchgeführt werden.

Die Herstellung der langkettigen Alkylglucoside erfolgt nach den oben zum Stand der Technik angegebenen Verfahren. Als reduzierend wirkende Monosaccharide kommen beispielsweise Pentosen oder Hexosen oder zu derartigen Monosacchariden hydrolysierbare Verbindungen in Betracht. Beispiele für geeignete Monosaccharide sind Glucose, Manose, Galaktose, Talose, Allose, Altrose, Idose, Arabinose, Xylose, Ribose und Lyxose sowie Mischungen aus den genannten Monosacchariden. Zu Monosacchariden hydrolysierbaren reduzierend wirkenden Zuckern gehören beispielsweise Oligosaccharide und Polysaccharide, wie Maltose, Lactose, Saccharose, Raffinose, Dextrine, Stärken, Maissirup und Holzzucker. Bevorzugt wird jedoch Glucose eingesetzt.

Die oben genannten Einsatzstoffe können in Gegenwart von üblichen sauren Katalysatoren direkt mit Alkoholen umgesetzt werden, die mindestens 8 C-Atome im Molekül aufweisen. Die langkettigen Alkohole enthalten üblicherweise 8 bis 32, vorzugsweise 12 bis 18 C-Atome. Bevorzugt werden technische Gemische von langkettigen Alkoholen eingesetzt, die aus der Oxo- und Ziegler-Synthese stammen. Technisch besonders gut zugänglich und daher auch für die Herstellung von langkettigen Alkylglucosiden bevorzugt, sind Mischungen aus $C_{10}/C_{16}$-, $C_{12}/C_{14}$ und $C_{12}$- bis $C_{18}$-Alkoholen.

Wie oben bereits erwähnt, ist es zur Herstellung langkettiger Alkylglucoside von Vorteil, zunächst niedrigkettige Alkylglucoside herzustellen, z.B. durch Umsetzung der Monosaccharide mit einem einwertigen $C_3$- bis $C_5$-Alkohol und die kurzkettigen Alkylglucoside dann durch Zusatz einwertiger langkettiger Alkohole einer Umacetalisierung zu unterwerfen. Die Herstellung der Glucoside und die Umacetalisierung erfolgen dabei jeweils in Gegenwart eines sauren Katalysators. Gemäß der Lehre der DE-OS 32 32 791 kann man bei der Herstellung der kurzkettigen Alkylglucoside in Gegenwart von beispielsweise Natriumperborat als Cokatalysator arbeiten. Bei allen oben genannten Verfahren entstehen Glucoside oder Glucosid-Gemische mit größeren Anteilen an unumgesetztem langkettigen Alkohol, weil ein Überschuß des Alkohols gegenüber dem Zucker bei der Reaktion wegen der sonst einsetzenden Selbstkondensation und des damit unerwünscht hohen Anteils an Oligosacchariden unumgänglich ist. Die, bezogen auf das Gewicht, verwendeten Mengen an langkettigem Alkohol liegen zwischen 1,2:1 bis 10:1 und betragen vorzugsweise 1,8:1 bis 2,0:1. Die jeweils anfallenden Reaktionsmischungen, die einen sauren Katalysator enthalten, werden durch Zusatz einer Base auf einen pH-Wert oberhalb von 6 eingestellt. Dieser pH-Wert wird dadurch bestimmt, daß man beispielsweise eine 50%-ige Lösung des Reaktionsgemisches in Wasser herstellt und die Messung bei einer Temperatur von 20° C vornimmt. Der pH-Wert des Reaktionsgemisches wird vorzugsweise auf Werte von 7,5 bis 9 eingestellt. Wichtig ist lediglich, daß pH-Werte im stark sauren Bereich vermieden werden, weil unter solchen Bedingungen bei den zur Extraktion nötigen Temperaturen eine teilweise Hydrolyse der Alkylglucoside eintritt.

Gemäß Erfindung werden die langkettigen Alkylglucoside mit Hilfe von Wasser aus den neutralisierten Reaktionsgemischen extrahiert.

Pro Volumenteil des Reaktionsgemisches benötigt man 0,5 bis 4, vorzugsweise 2 bis 3 Volumenteile Wasser. Vorzugsweise verwendet man entsalztes Wasser. Die Temperaturen der Reaktionsmischung bei der Extraktion betragen bis zu 100° C und liegen vorzugsweise in dem Bereich von 40 bis 60° C. Im allgemeinen führt man die Extraktion bei Temperaturen von 50 bis 55° C durch. Die Extraktion kann einmal oder mehrmals, z.B. zwei- bis viermal durchgeführt werden, indem man die oben angegebene Menge an Wasser in die entsprechenden Portionen aufteilt. Sofern eine kontinuierlich arbeitende Anlage zur Verfügung steht, kann die Extraktion auch kontinuierlich durchgeführt werden. Da sich bei der Behandlung des Reaktionsgemisches mit Wasser Emulsionen bilden können, empfiehlt sich bei kontinuierlichen Verfahren als Apparatur eine Mixer-Settler-Batterie. Sollte es bei der diskontinuierlichen Extraktion des Reaktionsgemisches zur Bildung von Emulsionen kommen, so können diese durch Zusatz üblicher Emulsionsspalter gebrochen werden.

Die aus der Reaktionsmischung gewonnen wäßrigen Alkylglucosidlösungen können entweder in Form der wäßrigen Lösung unmittelbar verwendet werden oder man destilliert daraus das Wasser ab, sofern man wasserfreie Alkylglucoside wünscht. Die bei der Extraktion mit Wasser anfallenden wäßrigen Alkylglucosidlösungen können jedoch auch noch einer weitergehenden Reinigung unterworfen werden, z.B. einer Extraktion mit einem organischen Lösemittel. Als organisches Lösemittel bieten sich beispielsweise die bei Herstellung des langkettigen Alkylglucosids aus der Reaktionsmischung abdestillierten kurzkettigen Alkohole an, sofern man die Alkylglucoside durch Transacetalisierung herstellt. Die als Lösungsmittel verwendbaren oder dem Lösungsmittel zusetzbaren kurzkettigen Alkohole haben neben ihrer Funktion als Lösemittel für die längerkettigen Alkohole noch eine emulsionshemmende und schaumdämpfende Wirkung. Die Behandlung der wäßrigen Alkylglucosidlösungen mit organischem Lösemittel, z.B. $C_2$-bis $C_6$-Alkoholen, ermöglicht eine weitere Reinigung der extrahierten wäßrigen Alkylglucosidlösungen von restlichen längerkettigen Alkoholen. Die mit Alkoholen behandelte wäßrige Alkylglucosidlösung wird noch kurze Zeit einer Wasserdampf-Destillation unterworfen, um daraus Restmengen an mitgeschleppten langkettigen Alkoholen zu entfernen, und die zur Reinigung eingesetzten kurzkettigen Alkohole azeotrop abzudestillieren.

Während man bei den bekannten Verfahren zur Reinigung von Reaktionsgemischen aus langkettigen Alkylglucosiden und langkettigen Alkoholen durch Destillation bei Drücken unterhalb von 1 mbar und Temperaturen bis zu 140° C arbeitet, reichen für das erfindungsgemäße Verfahren zur Reinigung Drücke bis herunter zu 50 mbar aus, so daß man ingesamt mit einem weitaus geringeren Investitionsaufwand für Anlagen auskommt. Bei der Herstellung der Alkylglucoside können auch die längerkettigen Alkohole, z.B. $C_{20}$- bis $C_{32}$-Alkohole ohne Schwierigkeiten für die nachfolgende Entfernung aus dem Reaktionsgemisch eingesetzt werden. Während nach den bekannten Verfahren zur Reinigung durch Destillation der Siedepunkt der langkettigen Alkohole und die Temperaturempfindlichkeit des langkettigen Alkylglucosids dem Reinigungsverfahren eine Grenze setzten, so daß besonders langkettige Alkohole, z.B. $C_{20}$- bis $C_{30}$-Alkohole für die Herstellung von Tensiden nicht in Betracht kamen, ist es nach dem erfindungsgemäßen Verfahren ohne Schwierigkeit möglich, auch diese langkettigen Alkylglucoside von den besonders langkettigen Alkoholen in einfacher Weise abzutrennen. Dadurch ist die Herstellung hydrophoberer langkettiger Alkylglucoside in reiner Form wesentlich vereinfacht.

Beispiel

Herstellung des langkettigen Alkylglucosids

In einem mit einem Rührer und einem Wasserauskreiser versehenen Reaktionsgefäß werden 1 500 ml (16 mol) n-Butanol, 855 g (4,5 mol) wasserfreie Dextrose, 200 ml Toluol und 7,5 g p-Toluolsulfonsäure eine Stunde lang zum Rückflußsieden erhitzt. Innerhalb dieser Zeit scheiden sich 24 ml (1,33 mol) Wasser ab. Man gibt dann 15 g Natriumperborat zu und kreist weitere 2 Stunden Wasser aus dem Reaktionsgemisch aus (32 ml entsprechend 1,8 mol). Anschließend werden bei einem Druck von 180 mbar 1 000 ml eines n-Butanol/Toluol-Gemisches abdestilliert. Man fügt dann 1 500 ml eines technischen Alkohol-Gemisches aus $C_{12}$-$C_{18}$-Alkoholen zu, erniedrigt den Druck auf 60 mbar und destilliert zunächst 2 Stunden bei einer Temperatur von 80° und danach innerhalb von 1 Stunde bei einer Sumpftemperatur von 100° das aus der Umacetalisierung entstehende n-Butanol ab.

Extraktion des Reaktionsgemisches

Das oben beschriebene Reaktionsgemisch wurde auf 60°C abgekühlt, mit 10 g Natriumacetat und 10 g Natriumcarbonat neutralisiert (pH 7,5). Um das darin enthaltene langkettige Alkylglucosid zu extrahieren, fügte man 2 l Wasser zu, rührte die Mischung 20 min bei 45°C und stellte dann den Rührer ab. Die Mischung trennte sich danach in zwei Phasen. Die untere Phase, die die Hauptmenge des langkettigen Alkylglucosids enthielt, wurde abgetrennt. Die Konzentration des Alkylglucosids betrug 26 Gew.% . Die obere abgetrennte Phase wurde anschließend mit 2 l Wasser und 200 ml des n-Butanol/Toluol-Gemisches versetzt, das bei der Herstellung des langkettigen Alkylglucosids bei der Umacetalisierung anfiel. Dieses Gemisch wurde 20 min bei 50°C gerührt. Danach ließ man es bei dieser Temperatur absitzen. Die untere wäßrige Phase wurde abgetrennt und die obere nochmals mit 2 l Wasser und 500 ml des abdestillierten n-Butanol/Toluol-Gemisches versetzt, bei 55°C 20 min lang gerührt und die Phasen getrennt. Die verbleibende obere Phase wurde azeotrop am Wasserabscheider vom emulgierten Wasser befreit und nach Ergänzung mit frischem langkettigen Alkohol bei der Herstellung von langkettigem Alkylglucosid eingesetzt.

Die bei der Extraktion erhaltenen wäßrigen Phasen wurden jeweils bei einem Druck von 50 mbar und einer Temperatur von 80°C eingeengt, dann einer Wasserdampfdestillation bei 80°C unterworfen (2 l Destillat) und durch Zusatz von 15 g Natriumperborat gebleicht. Durch Zugabe von Wasser wurde ein Feststoffgehalt von 50 Gew.% Alkylglucosid eingestellt. Man erhielt auf diese Weise 1 875 g einer wäßrigen Lösung eines langkettigen Alkylglucosids.

**Ansprüche**

1. Verfahren zur Reinigung von langkettigen Alkylglycosiden durch Extraktion von Reaktionsgemischen, die durch Umsetzung von Monosacchariden und mindestens 8 C-Atome enthaltenden Alkoholen oder durch Umacetalisierung von $C_1$- bis $C_6$-Alkylglucosiden mit mindestens 8 C-Atome aufweisenden Alkoholen erhältlich und die jeweils auf einen pH-Wert oberhalb von 6 eingestellt sind, dadurch gekennzeichnet, daß man die langkettigen Alkylglucoside mit Hilfe von Wasser aus den Reaktionsgemischen extrahiert.

2. Verfahren nach Anspruch 1, dadurch gekennzeichnet, daß man 1 Volumenteil der Reaktionsgemische mit 0,5 bis 4 Volumenteilen Wasser extrahiert.

3. Verfahren nach den Ansprüchen 1 und 2, dadurch gekennzeichnet, daß man Reaktionsgemische extrahiert, deren pH-Wert 7,5 bis 9 beträgt.

4. Verfahren nach den Ansprüchen 1 bis 3, dadurch gekennzeichnet, daß man die Reaktionsgemische 2 bis 4 mal oder kontinuierlich extrahiert.

5. Verfahren nach Anspruch 4, dadurch gekennzeichnet, daß man bei Temperaturen von 40 bis 60°C extrahiert.